# FASCICULE DE BREVET EUROPEEN

(11) **EP 2 704 852 B1**
(45) Date de publication et mention de la délivrance du brevet: **05.10.2016**
(21) Numéro de dépôt: 12725075.1
(22) Date de dépôt: 30.04.2012
(51) Int. Cl.: B05B 11/00, B05B 11/04, B65D 47/18, B65D 47/32, A61M 11/00

(54) **DISPOSITIF DE DISTRIBUTION DE LIQUIDE MUNI D'UN CANAL DE PASSAGE D'AIR**
MIT EINEM LUFTKANAL VERSEHENE FLÜSSIGKEITSAUSGABEVORRICHTUNG
LIQUID DISPENSING DEVICE EQUIPPED WITH AN AIR DUCT

(30) Priorité: 04.05.2011 FR 1153834
(43) Date de publication de la demande: 12.03.2014
(73) Titulaire: NEMERA LA VERPILLIERE, 38290 La Verpillière (FR)
(72) Inventeur: GREVIN, Guillaume, F-38080 l'Isle d'Abeau (FR); PAINCHAUD, Gaëtan, F-69340 Francheville (FR); DECOCK, Thierry, F-69002 Lyon (FR); JULIA, Xavier, F-38090 Villefontaine (FR)
(74) Mandataire: Vallée-Thiollier, Clémence-Olivia Laure Marie
(86) Numéro de dépôt international: PCT/FR2012/050962
(87) Numéro de publication internationale: WO 2012/150409

(56) Documents cités:
- EP-A1- 0 864 371
- WO-A1-98/48943
- FR-A1- 2 810 301
- US-A- 5 373 972
- US-A- 5 428 123
- US-A1- 2009 294 347

## Description

La présente invention concerne le domaine de la distribution de produit liquide, notamment sous forme de gouttes dans le domaine pharmaceutique, par exemple du liquide ophtalmique, nasal, buccal ou auriculaire. Plus particulièrement, l'invention concerne la distribution de liquide sans conservateur, en utilisant un réservoir déformable avec reprise d'air.

On entend par « produit liquide » un produit non solide et non gazeux. On comprend que le produit peut être plus ou moins liquide, selon sa viscosité, par exemple pâteux ou semi-liquide.

La tendance actuelle est de fournir des produits, notamment ophtalmiques, sans conservateur. Le dispositif de délivrance doit garantir la stérilité du produit tout au long de l'utilisation du flacon contenant le liquide à délivrer.

Selon un exemple, tel que décrit dans le document WO92/01625, un tel dispositif de distribution comprend un réservoir et un embout de distribution monté sur le réservoir, muni d'un orifice de distribution du liquide. L'utilisateur applique une pression sur le réservoir en le déformant et, sous l'effet de la pression, une goutte se forme à la sortie de l'orifice de distribution. Une fois la goutte délivrée, l'utilisateur relâche la pression et le réservoir tend à reprendre sa forme initiale, ce qui génère une dépression à l'intérieur du réservoir. Pour compenser cette dépression et permettre au réservoir de reprendre sa forme initiale, l'embout du dispositif comprend une entrée d'air dans le réservoir. Pour assurer que l'air entrant ne puisse pas contaminer le liquide contenu dans le réservoir, on positionne un filtre hydrophobe dans le passage d'air. Ce filtre évite l'entrée de microorganismes et de poussières, tout en empêchant la sortie du liquide. La porosité de ce type de filtre est en général de l'ordre de 0,2 micromètres, de telle sorte qu'elle empêche l'entrée de la plupart des microorganismes (par exemple, la bactérie Brevundimonas Diminuta qui a une taille de l'ordre de 0,2 micromètres).

Un problème de ce type de dispositif réside dans le fait qu'il est très difficile de garantir l'intégrité des filtres utilisés. En particulier, il est difficile de tester le bon fonctionnement du filtre après montage sur l'embout, car le filtre devrait alors être testé en présence d'eau ou de gaz, ce qui implique un risque de contamination ou de dégradation lors de la phase de test. Aucun de ces tests ne permet en outre de déceler de très petits défauts dans le filtre dans un laps de temps court, compatible avec des fabrications en très grands débits.

La présente invention a notamment pour but de proposer un dispositif de distribution de liquide avec reprise d'air garantissant de façon fiable la stérilité du contenu d'un réservoir.

A cet effet, l'invention a pour objet un dispositif de distribution de liquide conforme à la revendication 1.

On propose donc de réaliser la fonction de reprise d'air et de blocage de microorganismes de l'air, non pas en procédant à une filtration de l'air mais en utilisant les propriétés de diffusion de gaz de certains matériaux. Ainsi, on utilise un autre type d'organe qu'un filtre, à savoir un organe en matériau polymère non poreux. Un tel organe présente l'avantage d'assurer le passage d'air non contaminé de façon plus fiable qu'un filtre, lequel est poreux par définition. En effet, avec un organe non poreux, il est plus facile de tester qu'il n'y a pas de fuite due à un mauvais assemblage ou à un organe défectueux.

Par matériau « non poreux », on entend une matière pleine, sans trou, bloquant le passage de particules telles que des bactéries, par exemple bloquant la bactérie Brevundimonas Diminuta ayant une taille de l'ordre de 0,2 micromètres. Cet organe de diffusion se distingue d'un filtre. En effet, le matériau non poreux proposé pour l'organe de diffusion de l'air est composé d'un polymère utilisé dans sa forme brute, ayant subi par exemple une simple injection ou compression, alors qu'un matériau poreux tel que celui d'un filtre est composé d'un polymère ayant subi en outre des étapes de génération de pores ou interstices, telles qu'un étirement sur la matière ou une addition de solvant dans le polymère. Le matériau étant non poreux, il est étanche aux liquides et ne laisse pas passer de particules telles que des poussières ou des microorganismes. En revanche, ce matériau est perméable aux gaz, en particulier à l'air, du fait qu'il permet la diffusion de molécules de ces gaz. En d'autres termes, le matériau non poreux proposé ci-dessus a une perméabilité aux gaz qui laisse passer les molécules d'air, à travers un réseau, réticulé ou non, de chaines moléculaires enchevêtrées, de façon à laisser passer l'air par diffusion au travers de l'organe de diffusion.

On notera que, du fait que le matériau est non poreux, le passage d'air à travers l'organe est un processus plus lent qu'à travers un filtre, il peut prendre plusieurs minutes, voire plusieurs heures. Par exemple, pour un dispositif ayant permis de délivrer 6 gouttes, soit environ 240 µl de liquide, la dépression est quasiment compensée, c'est-à-dire que la pression interne du flacon est sensiblement équivalente à la pression externe, au bout de 12 heures. Le retour à une pression proche de la pression externe peut paraître long, mais les inventeurs à l'origine de l'invention ont constaté que ce n'était pas vraiment gênant, notamment quand le volume de liquide distribué à chaque administration est faible ou quand le temps s'écoulant entre deux administrations successives est long. C'est le cas, par exemple, de la distribution de gouttes de liquide, en particulier de gouttes de liquide ophtalmique.

On notera que, comme cet organe ne comporte pas de pores, il n'y a pas de risque de colmatage dû à une accumulation de microorganismes et de poussières dans les pores.

Ce type d'organe peut être testé de façon très facile après montage sur l'embout, sans contamination ou dégradation de l'organe. Par exemple, on peut appliquer une pression d'air d'un côté de l'organe et mesurer la pression de l'autre côté après quelques secondes. Comme le processus qui permet le rééquilibrage des pressions de chaque côté de l'organe est un processus qui prend plusieurs minutes voire plusieurs heures, et non quelques secondes, l'échelle de temps n'est pas la même que pour tester un filtre. Aussi, à l'échelle de la seconde, un organe non défectueux ne permettra pas de déceler de perte de pression, alors qu'on constatera une baisse de pression flagrante si l'organe est défectueux ou mal monté sur l'embout. De tels tests sont plus faciles, plus rapides et plus fiables que ceux effectués habituellement sur les filtres, qui risquent de les contaminer ou de les dégrader, ou alors qui sont statistiques, sur des échantillons détruits au cours du test, procurant une information relativement limitée.

On notera que l'organe de diffusion de l'air est de fabrication aisée et économique. Il se distingue ainsi du filtre hydrophobe ayant pour fonction de bloquer les microorganismes de l'air, ce filtre pouvant être coûteux à réaliser, d'une part pour assurer une filtration fine, d'autre part pour assurer son intégrité.

En outre, l'enveloppe d'isolation prévue autour de l'organe de diffusion du liquide permet d'éviter que le liquide à distribuer entre en contact avec l'organe de diffusion et qu'il puisse se produire des phénomènes de sorption de molécules du liquide sur l'organe de diffusion, c'est-à-dire les phénomènes, combinés ou non, d'absorption et d'adsorption d'une molécule dans ou sur une surface. Ces phénomènes de sorption sont d'autant plus critiques lorsque la concentration en principe actif dans la solution est faible, car ils ne permettent pas de garantir la délivrance de la quantité requise du principe actif au cours du temps. En effet, si le principe actif réagit, de façon réversible ou non, avec le matériau de l'organe de diffusion lorsqu'ils sont en contact, sa concentration dans la solution diminue et peut devenir trop faible, ce qui empêche la délivrance de la quantité attendue de médicament. Aussi, l'enveloppe d'isolation est particulièrement intéressante pour les liquides pour lesquels l'un des constituants peut interagir avec l'organe de diffusion de l'air et dans lesquels la concentration de principe actif dans la solution est inférieure à 1 % en poids, voire inférieure à 0,01 % , comme par exemple des solutions à base de prostaglandine ou d'un analogue de prostaglandine, destinées notamment au traitement de glaucomes.

On comprend que l'enveloppe d'isolation permet le passage de l'air de l'extérieur vers l'intérieur du réservoir, grâce à l'élément de passage d'air, et empêche le passage de liquide vers l'intérieur de l'enveloppe.

Le dispositif de distribution peut en outre comporter l'une ou plusieurs des caractéristiques suivantes prises seules ou en combinaison.
- L'élément de passage d'air est un filtre hydrophobe. Ce filtre n'a pas forcément besoin de présenter la fonction de blocage des particules présentes dans l'air entrant dans le réservoir, cette fonction étant assurée par l'organe de diffusion, mais uniquement la fonction d'empêcher le liquide de pénétrer dans l'enveloppe. On notera que, du fait de la présence de l'organe de diffusion, le filtre est de conception plus simple qu'un filtre hydrophobe habituellement utilisé, car il n'a alors pas besoin d'avoir une porosité aussi faible que 0,2 micromètres puisqu'il n'est pas destiné à empêcher la pénétration de microorganismes dans le réservoir. On peut par exemple utiliser un filtre ayant une porosité supérieure à 1 micromètres, par exemple voisine de 10 micromètres.
- L'élément de passage d'air est un clapet anti-retour. Ainsi, on peut prévoir un élément simple de passage d'air.
- L'enveloppe comporte une pièce de fixation de l'élément de passage d'air, cette pièce comportant une jupe cylindrique dans laquelle est rapportée une bague de serrage de l'élément de passage d'air contre la pièce de fixation. Cela permet d'assembler facilement l'élément de passage d'air, par pincement entre la pièce de fixation et la bague de serrage.
- L'enveloppe comporte un fond et une pièce de fixation de l'élément de passage d'air, cette pièce comportant une butée de positionnement de l'organe de diffusion contre le fond de l'enveloppe. On garantit ainsi que l'organe de diffusion est convenablement positionné dans le fond de l'enveloppe et donc que tout l'air entrant dans le dispositif passe, par diffusion, à travers l'organe de diffusion. La butée de positionnement est par exemple portée par une paroi annulaire de la pièce de fixation, s'introduisant dans un manchon délimitant le fond de l'enveloppe et venant pousser l'organe de diffusion contre ce fond.
- L'enveloppe comprend une surface extérieure délimitant, au moins en partie, un canal de passage de liquide. Le liquide n'entre donc pas en contact avec l'organe.
- Le canal de passage de liquide est un canal de réduction de débit. Ainsi, on peut créer des pertes de charge au sein du canal de passage de liquide, par exemple par des changements abrupts de direction et/ou de section, afin de mieux contrôler la qualité de la distribution, notamment en fonction de la viscosité du liquide et de la taille de gouttes que l'on souhaite délivrer.
- Le dispositif comporte un support dans lequel est réalisé le canal de passage d'air et sur lequel est rapporté l'enveloppe munie de l'organe de diffusion. Par exemple, le support comporte une cavité centrale tubulaire sur laquelle débouche le canal de passage d'air, à l'intérieur de laquelle est rapportée l'enveloppe munie de l'organe. De préférence, le support délimite, avec une surface extérieure de l'enveloppe, un canal de passage de liquide. Le support est par ailleurs, de façon avantageuse, un support pour une valve de distribution du liquide.
- L'organe de diffusion est muni d'une pluralité de reliefs, pour augmenter la surface de passage d'air par diffusion.
- Le matériau polymère de l'organe de diffusion comprend un élastomère à base de silicone. La perméabilité aux gaz de ce type d'élastomère permet de favoriser davantage le processus de reprise d'air.

On peut prévoir un procédé d'assemblage d'un dispositif tel que défini ci-dessus, dans lequel le dispositif comprend un support, et le procédé comporte une étape préalable d'assemblage de l'organe de diffusion dans l'enveloppe, puis une étape d'assemblage de l'enveloppe, munie de l'organe de diffusion, sur le support. Ce procédé d'assemblage est simple à mettre en oeuvre, notamment parce que l'assemblage préalable de l'organe dans l'enveloppe évite d'avoir à monter séparément l'organe de diffusion et l'élément de passage d'air sur le dispositif.

L'invention sera mieux comprise à la lecture de la description qui va suivre, donnée uniquement à titre d'exemple et faite en se référant aux dessins dans lesquels :
- la figure 1 est une vue schématique en coupe d'un dispositif de distribution de liquide selon un premier mode de réalisation ;
- la figure 2 est une vue explosée en perspective de l'enveloppe et de l'organe de diffusion du dispositif de la figure 1 ;
- la figure 3 est une vue explosée en perspective d'une enveloppe et d'un organe de diffusion d'un dispositif selon un deuxième mode de réalisation.

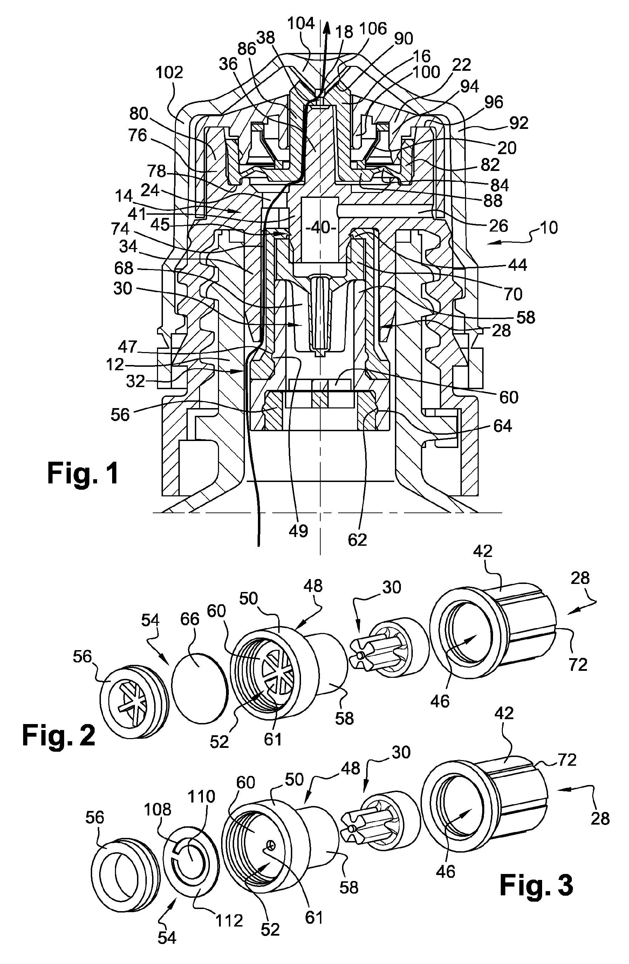

On a représenté sur à la figure 1 un embout 10 de distribution de liquide, sous forme de gouttes, destiné à être monté par vissage sur le col d'un réservoir 12. Ce réservoir 12 est un réservoir de stockage de liquide, par exemple du liquide pharmaceutique tel que du liquide ophtalmique. Le réservoir 12 est déformable de façon à distribuer du liquide par appui sur le réservoir. Plus précisément, la distribution de liquide se fait par pression, de la part d'un utilisateur, sur le corps du réservoir 12, ce dernier pouvant présenter une certaine élasticité pour reprendre sa forme initiale après la pression exercée par l'utilisateur ce qui génère une dépression à l'intérieur du réservoir 12.

L'embout de distribution 10 comprend, dans cet exemple, un support 14, une valve de distribution 16 munie d'un orifice de distribution 18, des moyens 20 de rappel de la valve 16 contre le support 14 en configuration de blocage, composés dans cet exemple d'une rondelle en matière plastique, et un capot 22. L'embout 10 définit un canal 24 de passage de liquide du réservoir 12 vers l'orifice de distribution 18, représenté par une flèche sur la figure1, et un canal 26 de passage d'air depuis l'extérieur vers l'intérieur du réservoir 12. Le canal 26 est obturé par un ensemble 28 comprenant un organe 30 de diffusion de l'air disposé dans une enveloppe 32 d'isolation de l'organe de diffusion 30.

Le support 14 comprend, dans cet exemple, une jupe intérieure 34 de forme tubulaire, s'étendant vers l'amont et permettant d'assurer l'étanchéité entre le réservoir 12 et l'embout de distribution 10, et une jupe extérieure de fixation sur le col 12 du réservoir, par exemple par vissage.

On comprend que l'on définit le sens amont et le sens aval en prenant comme référence le sens d'écoulement du liquide lors de sa distribution.

Le support 14 comporte par ailleurs une partie centrale d'étanchéité 36, de forme sensiblement cylindrique et s'étendant vers l'aval, opposée à la jupe 34. La partie 36 porte, sur son extrémité aval, une surface 38 d'appui de la valve 16 pour bloquer le passage de liquide, selon une configuration de blocage. Dans cet exemple, la surface d'appui 38 prend la forme d'un bourrelet annulaire.

Le support 14 délimite, dans cet exemple, le canal 26 de passage d'air vers l'intérieur du réservoir 12. Ce canal 26 qui débouche sur une cavité centrale 40 de forme sensiblement cylindrique, dont l'axe est confondu avec l'axe longitudinal du dispositif, et délimitée en partie par une paroi annulaire 41 du support. Cette cavité 40 reçoit, à son extrémité proximale, l'organe de diffusion 30. Le canal 26 de passage d'air est entièrement moulé dans le support 14. On notera que le canal 26 peut éventuellement comporter plusieurs sections de diamètre décroissant en partant de la périphérie du support 14 et en allant vers le centre du support 14 et ce, afin de faciliter les opérations et les outils de moulage.

La figure 2 représente une vue éclatée en perspective de l'ensemble 28. Dans ce mode de réalisation, l'enveloppe 32 comprend un manchon 42, de forme sensiblement tubulaire, muni d'un fond 44 comprenant un orifice central 45 sensiblement circulaire. Ce manchon 42 délimite une cavité 46 dans laquelle vient se loger l'organe de diffusion 30.

L'enveloppe 32 comprend également une pièce de fixation 48 comportant une paroi transversale 60 portant une première jupe tubulaire 50 s'étendant vers l'amont, délimitant avec la paroi 60 une cavité 52, et une deuxième jupe tubulaire 58 s'étendant vers l'aval, de diamètre inférieur à la première jupe 50. La paroi transversale 60 est percée d'au moins un orifice 61 permettant le passage de l'air.

La deuxième jupe 58 de la pièce de fixation 48 forme une butée de positionnement de l'organe de diffusion 30 contre le fond 44 du manchon 42. Ainsi, l'organe de diffusion 30 est pincé entre le fond 44 du manchon 42 et la deuxième jupe 58 de la pièce 48. La pièce de fixation 48 est maintenue en place à l'intérieur du manchon 42 par coopération d'un bourrelet annulaire 49 et d'une rainure annulaire 47, portés respectivement par une surface extérieure de la pièce de fixation 48 et une surface intérieure du manchon 42.

La cavité 52 de la pièce de fixation 48 reçoit un élément 54 de passage d'air, maintenu par serrage dans la cavité 52 par une bague de serrage 56 rapportée dans la première jupe 50. Le serrage de l'élément de passage d'air 54 est réalisé, dans le cas présent, par coopération d'un bourrelet annulaire 62 de la bague de serrage 56 dans une rainure annulaire 64 portée par la surface interne de la première jupe 50 de la pièce de fixation 48. Ce mode de serrage est simple et efficace. Toutefois, l'élément de passage d'air 54 pourrait également être collé, moulé ou soudé, par exemple par soudure par ultra-sons, sur la pièce de fixation 48.

Sur la figure 2, l'élément de passage d'air 54 est un filtre hydrophobe 66, qui permet le passage d'air mais bloque le passage de liquide. Cet élément 66 n'a pas de fonction de barrière anti-bactérienne ou anti-particules car cette fonction est assurée par l'organe de diffusion 30 de l'air. Ainsi, l'élément 66 a pour seules fonctions d'empêcher le passage de liquide vers l'organe de diffusion de l'air et de permettre à l'air de pénétrer dans le réservoir, il n'a pas, ou pas forcément, de fonction de blocage de microorganismes présents dans l'air extérieur. Le filtre 66 peut se présenter sous forme d'un disque de faible épaisseur.

L'organe de diffusion 30 est réalisé en matériau polymère perméable à l'air, ce matériau étant non poreux, ne laissant pas passer de particules ou de microorganismes (par exemple des bactéries de taille supérieure à 0,2 micromètres), mais laissant diffuser des molécules, telles que des molécules de gaz contenues dans l'air. Ainsi, le passage d'air à travers l'organe de diffusion 30 est réalisé par diffusion de gaz au travers de l'organe 30. Le matériau polymère comprend un matériau élastomère, à savoir, dans cet exemple, un élastomère à base de silicone, tel que du poly-di-méthyl-siloxane (PDMS). L'organe 30 a une forme générale cylindrique ou conique. il présente un axe central, confondu avec l'axe central de l'embout 10, cet axe correspondant à la direction de distribution de liquide. Plus précisément, l'organe 30 comprend une paroi 68 dite de diffusion de l'air, d'épaisseur relativement fine afin de favoriser l'échange de gaz, et une base comprenant une collerette annulaire 70 de fixation sur l'embout 10, cette collerette 70 ayant une épaisseur relativement importante, du moins supérieure à l'épaisseur générale de la paroi de diffusion 68.

La paroi de diffusion 68 comporte, dans cet exemple, une pluralité de reliefs qui permettent d'augmenter la surface d'échange d'air entre l'intérieur et l'extérieur du réservoir 12 sans pour autant augmenter considérablement l'encombrement de l'organe 30. Ces reliefs sont formés dans la paroi de façon qu'elle conserve son épaisseur relativement fine afin de laisser passer l'air. Les reliefs peuvent permettre en outre de rigidifier l'organe 30 et éviter ainsi d'utiliser de renforts. Dans cet exemple, les reliefs ont une section en forme de trèfle.

L'ensemble 28, comprenant l'enveloppe 32 et l'organe de diffusion 30, est monté d'un seul bloc par serrage mécanique de la collerette 70 et du manchon 42 sur la surface extérieure de la paroi annulaire 41 du support 14. Plus précisément, le diamètre intérieur de la collerette 70 est légèrement inférieur au diamètre extérieur de la paroi 41, de telle sorte que la collerette 70 est fixée sur la paroi 41 par serrage radial du fait de son élasticité. Éventuellement, on peut prévoir en outre des moyens de fixation mécanique de la collerette 70 sur la paroi 41, par exemple des moyens d'encliquetage tels qu'un bourrelet annulaire intérieur prévu sur la collerette 70 s'encliquetant dans une rainure annulaire prévue sur la surface extérieure de la paroi 41. L'orifice central 45 du manchon 42 a un diamètre intérieur légèrement inférieur au diamètre extérieur de la paroi 41, ce qui permet de fixer le manchon sur le support de manière étanche au liquide.

Grâce à cette enveloppe 32 délimitée par plusieurs pièces distinctes, à savoir le manchon 42, la pièce de fixation 48, l'élément de passage d'air 54 et la bague de fixation 56, l'organe de diffusion, une fois le dispositif de distribution de liquide 10 assemblé, est entièrement isolé du canal de passage de liquide 24. Il ne peut donc pas y avoir de phénomènes de sorption des molécules du liquide à distribuer sur l'organe de diffusion de l'air 30. On comprend que la paroi de diffusion 68 de l'organe présentant une surface relativement importante, il est avantageux d'éviter tout phénomène de sorption sur cette paroi 68. Ceci est d'autant plus important que la concentration en molécules du principe actif de la solution est faible, ce qui est le cas dans cet exemple, dans lequel le liquide distribué comprend des prostaglandines.

De plus, l'enveloppe 32 comprend une surface extérieure délimitant au moins un canal de passage de liquide 24. En outre dans cet exemple, le canal de passage de liquide 24 a également une fonction de limitation de débit de liquide. Plus précisément, le manchon 42 comprend, sur sa surface annulaire extérieure, une pluralité de gorges 72, représentées notamment sur les figures 1 à 3, et délimitant, avec la surface intérieure de la jupe 34 du support 14, des canaux 74 de réduction de débit de liquide. Ces canaux 74 ont un diamètre relativement restreint, de façon à diminuer la pression du liquide lorsque l'utilisateur appuie sur le réservoir 12. Selon une variante, les gorges 72 pourraient présenter des variations de direction ou de section, ou encore une forme hélicoïdale. Selon le nombre et la taille des gorges 72 placées en regard de la surface intérieure de la jupe 34, le débit de liquide pouvant sortir sera plus ou moins réduit.

Par ailleurs, le support 14 comprend une partie 76 de fixation de la valve de distribution 16 sur le support 14. Cette partie 76 fait également office de partie de fixation du capot 22 sur le support 14. Elle comprend une gorge annulaire 78 délimitée en périphérie par une paroi annulaire 80. La gorge annulaire 78 est par ailleurs délimitée, sur sa périphérie intérieure, par une nervure annulaire réalisée sur une paroi formant sensiblement un disque, traversée par le canal 24.

La valve de distribution 16 peut prendre une configuration de blocage et une configuration de passage du liquide, par coopération avec le support 14. Elle est réalisée, dans cet exemple, dans un matériau élastomère. Selon un autre exemple, seule une partie de la valve 16 est réalisée dans un matériau élastomère, l'autre partie étant réalisée dans un matériau plus rigide, pouvant servir d'appui aux moyens de rappel 20. La valve 16 comprend une partie 82 de fixation au support 14, formant une jupe de forme sensiblement tubulaire. Cette partie de fixation 82 est reliée à un voile 84 ayant sensiblement la forme d'un disque et à partir duquel une partie centrale 86 sensiblement cylindrique fait saillie. Le voile 84 comporte également un siège 88 d'appui des moyens de rappel 20. La partie 86 définit une cavité intérieure de forme sensiblement cylindrique, complémentaire de la partie 36. La partie 36 et la partie cylindrique 86 sont coaxiales et délimitent ensemble une partie du canal de passage de liquide 24. Le canal de passage de liquide 24 débouche sur l'orifice de distribution 18 réalisé dans l'extrémité aval de la valve 16, débouchant lui-même sur une forme 90 de formation de gouttes.

Le capot 22 comprend une partie 92 de fixation sur le support 14, annulaire, ainsi qu'une autre partie annulaire 94, coaxiale avec la partie 92, de façon à définir une gorge 96 dans laquelle la paroi annulaire 80 est encastrée. Le capot 22 comprend par ailleurs un siège 98 d'appui des moyens de rappel 20, prolongé sur sa périphérie intérieure par une paroi annulaire 100, traversée par la partie 86 et ayant une fonction de centrage de la partie 86 de la valve 16.

Le dispositif 10 comprend également un capuchon 102 amovible comprenant une partie 104 de forme sensiblement conique est complémentaire de la forme 90 de formation de gouttes. Cette partie 104 permet d'éviter que du liquide non distribué stagne dans la forme 90 lorsque le capuchon est monté sur le dispositif. En outre, la partie 104 comprend, sur sa surface destinée à être en contact avec la forme 90, une pluralité de gorges 106 qui favorisent l'évacuation, hors de la forme 90, du liquide pouvant y stagner, sans risque que le liquide puisse pénétrer à nouveau dans le dispositif.

Le fonctionnement du dispositif de la figure 1 va à présent être décrit.

Au repos, c'est-à-dire lorsque aucun utilisateur n'appuie pas sur le réservoir 12, la valve 16 est en configuration de blocage du liquide, c'est-à-dire qu'elle est en appui contre la surface 38, du fait de sa fixation permanente sur le support 14, exerçant une contrainte élastique sur la valve, et du fait de la pression effectuée par les moyens de rappel 20.

Lorsqu'un utilisateur appuie sur le réservoir 12, il exerce une pression sur le fluide qui s'écoule dans le canal de passage de liquide 24 et dans cet exemple, de réduction de débit 74, l'enveloppe 32 ne pouvant pas être traversée par un liquide. Au cours de son passage dans ce canal 74, le fluide voit son débit diminuer, par effet de perte de charge. Sous l'effet de la pression, le fluide déforme et soulève la valve 16 qui passe alors en configuration de passage de liquide, il s'écoule entre la valve 16 et la surface d'appui 38 et passe ensuite dans le canal 18 et dans la cavité 90 en se présentant sous forme de gouttes.

Une fois la goutte délivrée, l'utilisateur relâche la pression sur le réservoir 12 déformable, lequel tend à reprendre sa forme initiale, ce qui génère une dépression à l'intérieur du réservoir 12. La valve se ferme tout de suite sous l'action de la force de rappel des moyens de rappel 20 : il n'y a donc pas de liquide qui rentre à l'intérieur du dispositif. La dépression est compensée par une reprise d'air extérieur venant du canal de passage d'air 26 à travers l'organe de diffusion de l'air 30 perméable à l'air. On notera que, du fait que le matériau constituant l'organe 30 est non poreux, le passage d'air à travers l'organe 30 est un processus qui prend plusieurs minutes, voire plusieurs heures, et non quelques secondes.

Ainsi, si l'on prend comme exemple un dispositif d'une contenance de 12 ml, rempli de 10 ml d'une solution ophtalmique et muni d'un organe perméable à l'air comprenant du poly-di-méthyl-siloxane (PDMS) ayant une perméabilité à l'oxygène de 1,4*10⁻¹³mol*m⁻¹*Pa⁻¹*s⁻¹ (mol par mètre par Pascal et par seconde) et une surface d'échange de 90 mm² et une épaisseur de 0.4 mm, la délivrance de 6 gouttes de solution sous pression atmosphérique, c'est-à-dire de 40*6 = 240 microlitres de liquide, crée une dépression d'environ 95 mbar qui sera quasiment compensée en 12 heures (plus précisément, environ 90 mbar seront compensés au bout de 12 heures).

Étant donné que la paroi de l'organe 30 n'est pas poreuse, ce temps de reprise d'air dans le réservoir 12 est approximativement le même, que le dispositif soit en position « tête en bas » ou non. L'air peut ensuite passer à travers l'élément hydrophobe 66 afin de compenser la dépression créée au sein du dispositif par la délivrance d'une goutte de liquide. On comprend donc que le facteur limitant la reprise d'air est la perméabilité aux gaz de l'organe 30 et non l'élément hydrophobe 66.

On constate que grâce à l'enveloppe d'isolation 32, l'organe 30 n'entre pas en contact avec le liquide à distribuer.

On notera que l'organe 30, étant une pièce à part, peut avoir une forme qui varie en fonction des applications, des temps désirés de reprise d'air et/ou des réductions de débit souhaités. Il est donc possible de fabriquer des lots comprenant des embouts présentant une même valve 16, un même support 14, un même capot 18, mais présentant des organes 30 différents.

Le montage du dispositif de la figure 1 va à présent être décrit.

Selon une première étape d'assemblage de l'ensemble, l'organe de diffusion 30 est placé dans la cavité 46 du manchon 42, on insère ensuite la pièce de fixation 48, maintenue par encliquetage du bourrelet 49 avec la rainure 47. On place ensuite l'élément hydrophobe 66 dans la cavité 52 de la pièce de fixation 48 et on le pince grâce à la bague de fixation 56 encliquetée dans la première jupe 50.

On rapporte ensuite cet ensemble 28 sur le support 14 en l'emmanchant en force sur la paroi annulaire 41, selon une deuxième étape d'assemblage.

Grâce à la deuxième jupe 58 de la pièce de fixation 48, formant butée, on s'assure que l'organe de diffusion 30 est bien positionné dans la cavité 40 et que tout air venant de l'extérieur passe bien, par diffusion, à travers la paroi de diffusion 68 de l'air de l'organe 30 avant d'entrer dans le dispositif 10.

Dans le mode de réalisation de l'ensemble 28 représenté sur la figure 3, les éléments analogues à ceux du premier mode de réalisation sont identifiés par les mêmes références numériques.

On notera que dans ce mode de réalisation, l'élément de passage d'air 32 est un clapet anti-retour 108, ou valve anti-retour. Ce clapet 108 est, par exemple, formé par un disque central 110, en matière plastique, relié à un anneau 112 venu de moulage avec le disque 110. L'anneau 112 du clapet 108 est maintenu serré entre la paroi 60 de la pièce de fixation 48 et la bague de serrage 56. Le disque 110 prend, au repos, une configuration de blocage du liquide par coopération avec la paroi 60 et, lorsqu'il y a une dépression dans le réservoir 12, une configuration de passage d'air, dans laquelle le disque 110 ne coopère plus avec la paroi 60 pour obturer l'orifice 61 et permet à l'air pénétrant par diffusion à travers la paroi 68 de l'organe 30 d'entrer dans le réservoir. Plus précisément, le clapet 108 fonctionne avec une force de rappel élastique non nulle qui implique qu'il s'ouvre uniquement quand il existe une différence de pression entre son amont et son aval. Ainsi, quand la pression est identique de part et d'autre du clapet 108, celui-ci est plaqué contre son siège.

On notera que le clapet 108 peut prendre bien d'autres formes que celle illustrée sur la figure 3. Tout type de clapet (ou valve) anti-retour peut être envisagé, en particulier un clapet dont la fermeture est assurée par l'élasticité d'un matériau (par exemple un clapet de type bec de canard, souvent appelé "duck-bill") et/ou par la force de rappel d'un ressort (par exemple un clapet sous forme d'un pointeau ou d'un disque rappelé par un ressort).

Le reste du dispositif de la figure 3 est analogue à celui des figures précédentes.

On notera que l'invention n'est pas limitée aux modes de réalisation précédemment décrits.

Notamment, l'organe de diffusion de l'air 30 peut prendre une forme différente de celle présentée sur les figures. Il pourrait, par exemple, comprendre une paroi 68 de forme cylindrique ou conique, présentant un sommet obturé par une surface en forme de disque et une collerette annulaire 70 de fixation sur l'embout 10. La paroi pourrait comporter en outre, afin d'être rigidifiée, des nervures de renfort, correspondant à des augmentations locales de l'épaisseur de la paroi 68. Dans une autre variante, la paroi de diffusion 68 pourrait comporter, à la place ou en plus des nervures de renfort, une pluralité de reliefs qui permettent d'augmenter la surface d'échange d'air entre l'intérieur et l'extérieur du réservoir 12 sans pour autant augmenter considérablement l'encombrement de l'organe 30. Ces reliefs sont formés dans la paroi de façon qu'elle conserve son épaisseur relativement fine afin de laisser passer l'air.

Les moyens de rappel de la valve 16 contre son support ne sont pas indispensables et ne sont pas limités à une rondelle ressort 20 comme représenté sur la figure 1. On peut également envisager d'utiliser, par exemple, un ressort.

## Revendications

1. Dispositif (10) de distribution de liquide comportant :
- un canal de passage d'air (26) depuis l'extérieur vers l'intérieur d'un réservoir (12) de liquide,
le dispositif étant **caractérisé en ce qu'**il comporte en outre :
- un organe d'obturation (30) du canal de passage d'air (26), dit organe de diffusion de l'air, réalisé en matériau polymère perméable à l'air, non poreux, apte à bloquer des particules ayant une taille de l'ordre de 0.2 micromètres et n'ayant pas subi une étape de génération de pores ou d'interstices, et
- une enveloppe d'isolation (32) de l'organe de diffusion (30), configurée de telle sorte que l'organe de diffusion (30) ne soit pas en contact avec le liquide du réservoir, l'enveloppe (32) comprenant un élément de passage d'air (54) vers l'intérieur du réservoir (12).

2. Dispositif (10) selon la revendication précédente, dans lequel l'élément de passage d'air (54) est un filtre hydrophobe (66) ou un clapet anti-retour (108).

3. Dispositif (10) selon l'une quelconque des revendications précédentes, dans lequel l'enveloppe (32) comporte une pièce de fixation (48) de l'élément de passage d'air (54), cette pièce (48) comportant une jupe cylindrique (50) dans laquelle est rapportée une bague de serrage (56) de l'élément de passage d'air (54) contre la pièce de fixation (48).

4. Dispositif (10) selon l'une quelconque des revendications précédentes, dans lequel l'enveloppe (32) comporte un fond (44) et une pièce de fixation (48) de l'élément de passage d'air (54), cette pièce (48) comportant une butée de positionnement (58) de l'organe de diffusion (30) contre le fond (44) de l'enveloppe (32).

5. Dispositif (10) selon l'une quelconque des revendications précédentes, dans lequel l'enveloppe (32) comprend une surface extérieure délimitant un canal de passage de liquide (24).

6. Dispositif (10) selon la revendication précédente, dans lequel le canal de passage de liquide (24) est un canal de réduction de débit (74).

7. Dispositif (10) selon l'une quelconque des revendications précédentes, comportant un support (14) dans lequel est réalisé le canal de passage d'air (26) et sur lequel est rapporté l'enveloppe (32) munie de l'organe de diffusion (30).

8. Dispositif (10) selon l'une quelconque des revendications précédentes, dans lequel l'organe de diffusion (30) est muni d'une pluralité de reliefs.

9. Dispositif (10) selon l'une quelconque des revendications précédentes, dans lequel le matériau polymère de l'organe de diffusion (30) comprend un élastomère à base de silicone.

## Patentansprüche

1. Vorrichtung (10) zum Ausgeben von Flüssigkeit, die Folgendes aufweist:
- einen Kanal für den Durchgang von Luft (26) von außerhalb nach innerhalb eines Flüssigkeitsbehälters (12),
wobei die Vorrichtung **dadurch gekennzeichnet ist, dass** sie ferner Folgendes aufweist:
- ein Organ zum Verschließen (30) des Luftdurchgangskanals (26), das so genannte Luft-Ausgabeorgan, das aus einem nicht porösen, luftdurchlässigen Polymermaterial realisiert ist und dazu eingerichtet ist, Partikel mit einer Größe in der Größenordnung von 0,2 Mikrometer zu blockieren, die nicht einem Schritt der Erzeugung von Poren oder von Zwischenräumen unterzogen wurden, und
- eine Umhüllung zum Isolieren (32) des Ausgabeorgans (30), die so ausgebildet ist, dass das Ausgabeorgan (30) mit der Flüssigkeit des Behälters nicht in Kontakt ist, wobei die Umhüllung (32) ein Element für den Durchgang von Luft (54) nach innerhalb des Behälters (12) umfasst.

2. Vorrichtung (10) nach dem vorhergehenden Anspruch, wobei das Luftdurchgangselement (54) ein hydrophober Filter (66) oder ein Rückschlagventil (108) ist.

3. Vorrichtung (10) nach einem der vorhergehenden Ansprüche, wobei die Umhüllung (32) ein Teil zum Befestigen (48) des Luftdurchgangselements (54) aufweist, wobei dieses Teil (48) eine zylindrische Schürze (50) aufweist, in der ein Ring zum Festklemmen (56) des Luftdurchgangselements (54) gegen das Befestigungsteil (48) angebracht ist.

4. Vorrichtung (10) nach einem der vorhergehenden Ansprüche, wobei die Umhüllung (32) einen Boden (44) und ein Teil zum Befestigen (48) des Luftdurchgangselements (54) aufweist, wobei dieses Teil (48) einen Anschlag zum Positionieren (58) des Ausgabeorgans (30) gegen den Boden (44) der Umhüllung (32) aufweist.

5. Vorrichtung (10) nach einem der vorhergehenden Ansprüche, wobei die Umhüllung (32) einen Außenfläche umfasst, die einen Flüssigkeitsdurchgangskanal (24) begrenzt.

6. Vorrichtung (10) nach dem vorhergehenden Anspruch, wobei der Flüssigkeitsdurchgangskanal (24) ein Durchflussbegrenzungskanal (74) ist.

7. Vorrichtung (10) nach einem der vorhergehenden Ansprüche, die einen Träger (14) aufweist, in dem der Luftdurchgangskanal (26) realisiert ist und an dem die mit dem Ausgabeorgan (30) versehene Umhüllung (32) angebracht ist.

8. Vorrichtung (10) nach einem der vorhergehenden Ansprüche, wobei das Ausgabeorgan (30) mit einer Vielzahl von Erhöhungen versehen ist.

9. Vorrichtung (10) nach einem der vorhergehenden Ansprüche, wobei das Polymermaterial des Ausgabeorgans (30) ein Elastomer auf Silikonbasis enthält.

## Claims

1. A liquid dispenser device (10), comprising:
· an air flow channel (26) for passing air from the outside towards the inside of a liquid reservoir (12);
the device being **characterized in that** it also comprises:
· a closure member (30) for closing the air flow channel (26), said closure member being referred to as an "air diffuser member" and being made out of a non-porous polymer material that is permeable to air, able to block particles having a size of about 0.2 micrometers and having not been subjected to a step of generating pores or interstices; and
· an isolator casing (32) for isolating the diffuser member (30), said isolator casing being configured in such a manner that the diffuser member (30) is not in contact with the liquid from the reservoir, the casing (32) including an air flow element (54) for passing air towards the inside of the reservoir (12).

2. A device (10) according to the preceding claim, wherein the air flow element (54) is a hydrophobic filter (66) or a check valve (108).

3. A device (10) according to either preceding claim, wherein the casing (32) includes a fastener piece (48) for fastening the air flow element (54), the piece (48) including a cylindrical skirt (50) in which there is fitted a clamping ring (56) for clamping the air flow element (54) against the fastener piece (48).

4. A device (10) according to any preceding claim, wherein the casing (32) includes an end wall (44) and a fastener piece (48) for fastening the air flow element (54), the piece (48) including a positioner abutment (58) for positioning the diffuser member (30) against the end wall (44) of the casing (32).

5. A device (10) according to any preceding claim, wherein the casing (32) includes an outer surface that defines a liquid flow channel (24).

6. A device (10) according to any preceding claim, wherein the liquid flow channel (24) is a flowrate reducer channel (74).

7. A device (10) according to any preceding claim, including a support (14) in which the air flow channel (26) is formed, and in which the casing (32) provided with the diffuser member (30) is fitted.

8. A device (10) according to any preceding claim, wherein the diffuser member (30) is provided with a plurality of portions in relief.

9. A device (10) according to any preceding claim, wherein the polymer material of the diffuser member (30) comprises a silicone rubber based elastomer.
